Europäisches Patentamt

**European Patent Office**

Office européen des brevets

(11) Publication number: **0 291 046**

**A1**

## EUROPEAN PATENT APPLICATION

(21) Application number: **88107612.9**

(22) Date of filing: **11.05.88**

(51) Int. Cl.⁴: **G01N 33/80 , G01N 33/564**

(30) Priority: **12.05.87 IL 82501**
**28.04.88 IL 86215**

(43) Date of publication of application:
**17.11.88 Bulletin 88/46**

(84) Designated Contracting States:
**AT BE CH DE ES FR GB GR IT LI LU NL SE**

(71) Applicant: **YEDA RESEARCH AND**
**DEVELOPMENT COMPANY, LTD.**
**Kiryat Weizman P.O. Box 95**
**Rehovot 76100(IL)**

(72) Inventor: **Cohen, Irun Robert**
**11 Hankin Street**
**Rehovot(IL)**

(74) Representative: **Vossius & Partner**
**Siebertstrasse 4 P.O. Box 86 07 67**
**D-8000 München 86(DE)**

(54) **Monitoring of efficacy of vaccination.**

(57) The invention relates to an assay for determining the efficacy of vaccination of a patient in need thereof against an autoimmune disease and to means for carrying out such assay.

Vaccinations are carried out by means of activated treated T cells, or membranes of these or fragments of such membranes. The assay according to the invention is in the form of a skin test or in the form of a proliferative assay in vitro carried out on peripheral leucocytes of the patient which determines either one or both of the anti-idiotypic T cell responses or the anti-activated indicator cell response.

EP 0 291 046 A1

## MONITORING OF EFFICACY OF VACCINATION

The invention relates to a method (assay) for monitoring the efficacy of vaccinations against autoimmune diseases and to means for carrying out such monitoring assays. Such vaccinations can be effected as disclosed in a number of publications and patents, they are generally based on the use of certain T cells. Especially effective are vaccinations with either pressure-treated T cells, with surface-membranes shed from T cells, with cross-linked surface-proteins from activated T cells, with T cells treated with certain cross-linkers, T cells treated with certain mitogens etc. Such assay determines the efficacy of vaccination by a skin test or by an in vitro assay, of either one or both the "anti-idiotype" response and the "anti-activation indicator" response of T cells of the patient. A detailed explanation of these will be given in the following.

It is of importance to determine the efficacy of vaccination so as to know whether a repetition is required and what doses have to be administered for an effective vaccination.

The present invention provides simple means for carrying out such assays.

The invention relates to an assay for monitoring and for establishing quantitative parameters of the effectivness of vaccination against autoimmune diseases. The invention furthermore relates to means and kits for carrying out such assays.

It is possible to vaccinate patients against certain types of autoimmune diseases. Such vaccinations can be carried out with a variety of T cells. Amongst others there may be used pressure-treated T cells. There may be used T cells activated by incubation with mitogens or specific antigens. There may be used T cells treated with a cross-linking agent. There may be used certain surface proteins shed from the surface of T cells after pressure treatment. There may be used cross-linked cell membranes etc.

Although the inventor does not want to be bound by any specific scientific theory, it is believed that the activated T cell used for immunization comprises two types of sites: (a) an antigen receptor site (the idiotype); (b) activation indicator sites. The latter, (b) are present only in activated T cells.

When such cells are used for vaccination, the efficacy of the vaccination can be assayed either by an in vivo skin test, or by an in vitro test. Such test determines the development of T cells of the patient responding to one or both (a) and (b): The response to (a) being designated as "anti-idiotypic response"; the response to (b) being designated hereinafter as "anti-activated site response".

The patient may develop either response (a) or (b) or both, and each of them, or their combination may be assayed, the results being indicative of the efficacy of the vaccination.

As stated above, the vaccination can be carried out with either activated T cells, with membranes of these, or with membrane fragments of these. In a similar manner the assays can be carried out with any of these.

Thus, the anti-idiotypic response may be determined by means of a T cell membrane thereof or membrane fraction thereof bearing the idiotype of the vaccine.

The activation sites may be assayed by means of activated T cells (membranes or fragments of these) derived from the patient or any other source, irrespective of the idiotype. The assays for the invention can be carried out in different manners.

The monitoring of the efficacy of the T cell vaccination can be effected by determining the augmentation of a T cell response specific to the T cell vaccine, or to the antigen receptors or other membrane components of the T cell vaccine. This response may be termed anti-idiotypic because it is directed against the unique idiotypes of the T cells used for vaccination. It is generally held that the idiotypes of T and B lymphocytes are determined by the nature of the antigen receptors of the lymphocytes.

In addition to the detection of anti-idiotypic T cell responses, the success of vaccination can be detected by an increase in T cell responsiveness to markers of T cells related to the state of activation of the T cells irrespective of their idiotypes (anti-activated cells). It was shown that successful vaccination requires that the vaccinating T cells be activated prior to administration by incubation for about 48 hours of the T cells with their specific antigen or with a T cell mitogen such as Concanavalin A (Con A) or phytohemagglutinin (PHA) (for example see Y. Naparstek, et al., Eur. J. Immunol. 13 418-423(1983)). T cells that have not been activated by antigen or mitogen do not vaccinate, despite the fact that they have antigen receptors, i.e., idiotypes. This has led to the conclusion that the signal for resistance to the autoimmune disease borne by the vaccinating T cells includes both an idiotype (the T cell antigen receptor) and an element induced by activation of the T cell. The activation indicator molecule has not yet been identified, but it may be inferred that the required "activation indicator" is anchored in the T cell membrane because isolated membranes of activated T cells may be used to vaccinate, provided that the membranes have been treated with a chemical cross-linker such as glutaraldehyde, formaldehyde, or 8-methoxy-psoralen and ultraviolet A light. Several membrane-associated markers of T cell activation have been identified such as the IL-2 receptor, class II molecules of the major histocompatibility complex (MHC), or the CD-2 complex of molecules. The activation marker required for vaccination might be any of these, or other molecules. In any case, the discovery forming the basis of the present invention is that successful T cell vaccination leads to two separate T cell responses: an anti-idiotypic response which is detectible by employing as test reagents either activated or non-activated T cells bearing the idiotypes of the T cell vaccine, and an anti-activated T cell response employing as a test reagent activated T cells of any idiotype. The latter reagent detects T cell responses directed to the "activation indicator" (markers). These activation molecules are relatively deficient or are not expressed by non-activated T cells. There can be used in vitro proliferation or delayed type hypersensitivity (DTH) skin tests.

An in vivo skin test is advantageously carried out about 7 to 10 days after vaccination, and at regular intervals thereafter. T cells or membranes or molecules of the type suitable for vaccination are suspended in saline, about 100,000 to $10^7$ cells/ml and 0.2 ml of such a suspension are injected intracutaneously and induration and redness are measured after 24 to 48 h. A reddened area of greater than 3 to 5 mm diameter is indicative of an effective vaccination.

The same procedure may be carried out using membranes of said cells or fragments thereof obtained from an equivalent number of cells. For such tests there may be used a quantity of 10 to 500 ug/ml of membrane proteins of the type used for such vaccinations.

Another embodiment of the invention is an in vitro assay and this comprises withdrawing peripheral blood, separating the white cells by standard procedures, suspending the white cells in a suitable RPMI medium in 5% human AB serum and antibiotics, at a concentration of about 500,000 to $10^7$ cells/ml with irradiated stimulator cells at a ratio of stimulators to responders of between 1:1 and 10:1. The anti-idiotypic response can be measured by itself without the anti-activated cell response by using non-activated stimulator cells bearing the idiotypes of the T cell vaccine. If the stimulator cells bearing the vaccine idiotype have been activated by antigen or a mitogen, then the response will measure a combination of the anti-idiotype and the anti-activated cell responses. If the stimulator cells do not bear the vaccine idiotype and have been activated, then the anti-activated cell response alone will be detected. Table 1 illustrates this schematically.

Stimulator cells may be fixed with chemical cross-linkers such as glutaraldehyde, formaldehyde, or 8-methoxy-psoralen and ultraviolet A, or membrane fragments of these cells may be used. Evaluation is made after culture for about 5 to 7 days and there is measured by standard means the incorporation of tritiated thymidine. As control and "background noise" there is used a culture of the non-activated peripheral blood cells of the patient without any addition: the actual measurement is considered positive if the incorporation exceeds by a factor of 2 or more the incorporation of the control cells.

There may be provided kits for carrying out such assays, which comprise the effective ingredients in a form suitable for prolonged storage, diluting means and means for evaluating the results of the measurements. The aforementioned effective ingredients are for instance suitably treated whole cells, cell membranes or fragments of cell membranes which may be prepared according to known methods, or defined molecules obtained from cells or by genetic engineering. The invention is illustrated in the following with reference to results obtained with laboratory animals. The results demonstrate the applicability of the assay to human autoimmune diseases.

The following examples are to be construed in a non-limitative manner.

Anti-T cell responses in vaccinated rats:

Rats vaccinated with suitable T cells against adjuvant arthritis (AA) or experimental autoimmune encephalomyelitis (EAE) develop responding T cells that recognize the antigen receptors of the vaccinating T Cell (anti-idiotypic T cell immunity), and responding T cells that recognize activated T cells that do not bear the specific idiotype of the T cell vaccine (anti-activation indicator immunity).

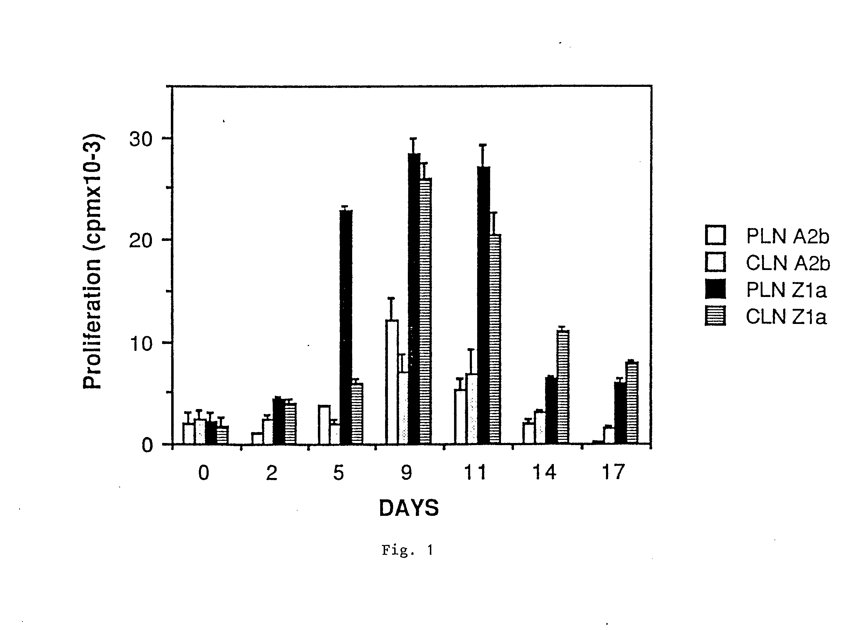

Figure 1, demonstrates the proliferation response of rats vaccinated in the hind footpad against EAE using $10^4$ activated cells of the Z1a clone as a vaccine. The responses to clone Z1a or to clone A2b (a control clone negative for the Z1a idiotype that is not associated with EAE) on various days after vaccination were measured in the popliteal lymph node (PLN) draining the site of vaccination and in the cervical lymph node (CLN; indicating systemic immunity). It can be seen that on day 5 there was a specific (anti-idiotypic) proliferative response to the Z1a vaccine in the PLN. By day 9, the anti-idiotypic response was systemic - both in the PLN and CLN. The anti-idiotypic responses waned, but still remained positive on day 17.

An anti-activation indicator response to clone A2b (negative for the Z1a idiotype) may be seen on days 9 and 11. These responses, although less than the anti-idiotypic response to Z1a, were significantly greater than the background responses shown by the control, non-vaccinated rat T cells (day 0). The response to non-activated clone A2b on days 9 and 11 was the same as the control (not shown). Thus, the response to idiotype-negative A2b required that A2b be activated, defining the response as an anti-activated T cell response.

Vaccination against AA using a low dose of A2b led to a specific anti-idiotypic response to non-activated clone A2b and not to non-activated clone Z1a (not shown). Therefore, the anti-idiotypic response was related to the specificity of the vaccination. Vaccination with activated A2b also led to an anti-activated T cell response to activated Z1a (see below).

## Anti-idiotypic response to T cell vaccination involves both helper and suppressor T cells:

To analyse the cellular components of the anti-idiotypic response to vaccination, we cloned the anti-idiotypic T cells from the PLN of Z1a vaccinated rats and studied their properties. Anti-idiotypic clones of two types were detected: $CD4^+8^-$ helper T cells, and $CD4^-8^+$ suppressor T cells. To study the effects of these cells on clone Z1a, we irradiated the anti-idiotypic clones and cultured them with Z1a. Table 2 shows that the helper clone, TR1, stimulated clone Z1a while the suppressor clone TR8 suppressed the response of Z1a to its antigen. TR1 and TR8 did not stimulate or suppress the control clone A2b.

## Anti-activated cell response to vaccination involves both helper and suppressor T cells:

To analyze the components of the anti-activated cell response, we vaccinated rats in the hind footpads with clone A2b and 6 days later stimulated in vitro the popliteal lymph node cells with activated clone D9, which in a subclone of Z1a that does not share the A2b idiotype. The population of lymphocytes responding to the activated cell markers of Z1a were found to possess both $CD4^+$ "helper" T cell marker (65%) and the $CD8^+$ "suppressor" T cells (35%). Therefore, similar to the anti-idiotypic T cells, the anti-activated T cells include both helper and suppressor types.

## Anti-idiotypic skin tests:

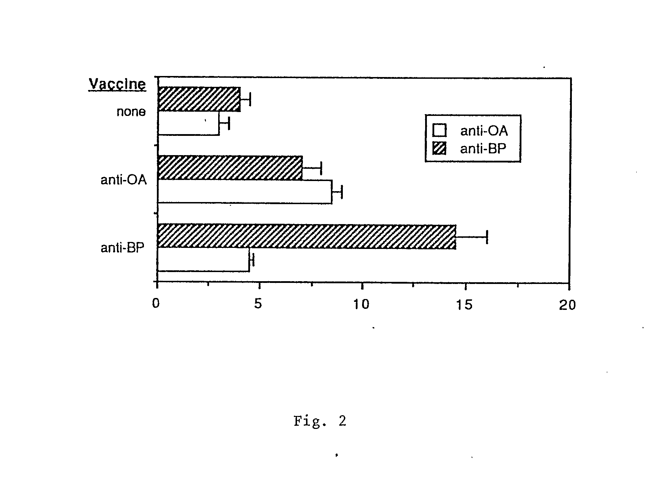

In addition to anti-idiotypic T cell proliferation in vitro, vaccinated rats manifested anti-idiotypic skin tests to the vaccinating T-cells. Figure 2 shows that rats vaccinated with $10^4$ anti-BP clone (Z1a) responsed to Z1a in a delayed type hypersensitivity (DTH) test, and not to a control anti-ovalbumin (OA) clone.

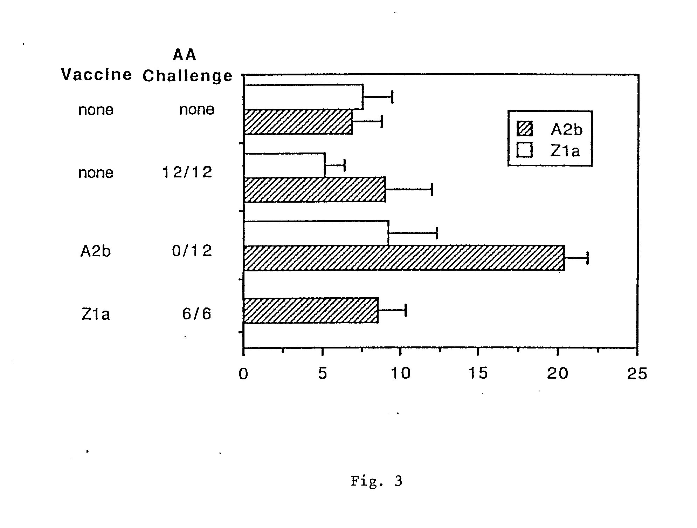

Figure 3 shows that rats vaccinated with pressure treated clone A2b responded in a DTH skin test to clone A2b, but not to clone Z1a. Thus, pressure treated T cells also induce an anti-idiotypic response

## No anti-idiotypic DTH following inadequate vaccination:

Table 3 shows that only adequate vaccination produces an anti-idiotypic response. Rats were vaccinated with clone A2b treated either with gamma-irradiation (ineffective vaccine) or with the chemical cross-linker glutaraldehyde (effective vaccine). The rats were then tested for DTH reactivity to clone A2b. Only vaccination with the glutaraldehyde treated A2b induced an anti-A2b DTH test. As glutaraldehyde treated, but not gamma-irradiated A2b successfully vaccinates against AA, we can conlcude that the anti-idiotypic DTH response was associated with effective vaccination.

Anti-activated indicator response induced by intense vaccination:

To induce a strong response Lewis rats were vaccinated weekly for 3 weeks with 2 x $10^7$ Concanavalin A activated A2b cells, treated with glutaraldehyde as described (Lider O. et al., Proc. Natl. Acad. Sci. U.S.A., 84 4577-4580 (1987)). One week later the rats were assayed for their DTH response to activated or non-activated clone A2b (idiotype specific) or clone D9 (a subclone of Z1a lacking the A2b idiotype).

It can be seen in Table 4 that the non-activated T clone elicited a specific anti-idiotypic response: the response to non-activated A2b was 4 fold higher than the response to non-activated D9. In contrast, both activated clones elicited a strong response that was not statistically different. Thus, the activated T cells elicited the anti-activated indicator response, while the non-activated T cells elicited the anti-iodiotypic response.

The results tabulated in Table 4 show that the anti-activated indicator response was relatively stronger than the anti-idiotypic response. This contrasts with the results presented in Figures 1 and 2 in which the anti-idiotypic response was stronger than the anti-activated indicator response. The difference between the experiment in Table 4 and the other experiments may be attributed to the fact that the vaccination procedure in Figures 1 and 2 employed a single inoculation of $10^4$ activated T cells, while the vaccination procedure in Table 4 employed a much greater number (2 x $10^7$ given 3 times) of glutaraldehyde treated T cells. Thus a more vigorous vaccination with multiple doses of cross-linked T cells may make the anti-activated indicator response more prominent. The experiment in Figure 3 shows that pressure treated A2b T cells produced a prominent anti-idiotypic indicator response.

Clinical use of anti-idiotypic response:

The effectiveness of T cell vaccination of patients is monitored by testing the patient for an anti-idiotypic T cell response to the vaccine, or for an anti-activated cell response to activated T cells. For example, vaccines prepared as described in U.S. Patent No. 4,634,590, European Patent Application No. 87113856.6 and Lider O. Proc. Natl. Acad. Sci. USA, vol 84, pp. 4577-4580, July 1987, can be used to vaccinate persons against type I diabetes, rheumatoid arthritis, multiple sclerosis, or other autoimmune conditions. Ten days later and at determined intervals thereafter, peripheral blood leukocytes are tested in a proliferative assay against the patient's cells used for vaccination to test anti-idiotypic response, or against activated T cells not of the vaccine idiotype to test for anti-activated cell response, or the patient is skin tested using the vaccine. The degree of response indicates the degree to which the patient has responded to the vaccine. A weak response suggests that the patient should receive further vaccination. The patients can be tested at a later date to ascertain whether protection has waned and a booster vaccination is required.

The anti-idiotypic proliferation or DTH responses can be done using whole T cells or membrane fragments of T cells containing the T cell receptor prepared with hydrostatic pressure as described in the Cohen-Shinitzy Patent/Patent applications/publications ibid. or by any other means. Membrane fragments/components of activated T cells can be used to assay the anti-activated cell response.

In a similar manner membranes of such T cells treated with chemical cross-linkers or fragments of such T cells can be used for either vaccination or for the assay, giving similar results.

According to the invention there are also provided kits comprising the required components in a form suitable for prolonged storage. Furthermore the invention relates to kits where the components are provided in unit dosage form. Such kits also comprise for one of the embodiments, suitable cells stored with preservatives and means for detecting the interaction of patients with the test cells.

The Figures show:

Figure 1 illustrates anti-idiotype and anti-activated site T cell response;

Figure 2 illustrates the results of anti-idiotypic skin test reactivity following T cell vaccination against EAE;

Figure 3 illustrates anti-idiotypic skin test reactivity following T cell vaccination against AA.

As stated above, Figure 1 demonstrates the anti-idiotypic and anti-activated cell T cell response. Lewis rats were vaccinated against EAE by immunization in the hind footpads to $10^4$ clone Z1a cells. At the indicated days, the draining popliteal lymph nodes (PLN) and distal cervical lymph nodes (CLN) were removed and 5 x $10^5$ cells were tested in vitro for a T cell proliferative response to $10^6$ irradiated Z1a (idiotype positive) or A2b (idiotype negative) clones that had been activated. Proliferation was measured by the incorporation of tritiated thymidine into DNA.

Figure 2. demonstrates the anti-idiotypic skin test reactivity following T cell vaccination against EAE. Lewis rats were vaccinated against EAE by vaccination with $10^4$ anti-BP clone Z1a cells. Control rats were vaccinated with $10^4$ T cells reactive to a foreign antigen ovalbumin (anti-OA). Nine days later the rats were tested for delayted type hypersensitivity to an ear challenge with $10^5$ irradiated anti-BP or anti-OA clone cells. Ear swelling was measured 48 hrs later. Histologic examination of the positive ears showed evidence of DTH reaction., and Figure 3 demonstrates anti-idiotypic skin test reactivity following T cell vaccination against AA. Lewis rats were vaccinated against AA by immunization with $2 \times 10^7$ clone A2b treated with hydrostatic pressure (1250 bars, 15 minutes). Two weeks later the rats were challenged with MT to induce AA. Four weeks later the rats were challenged with irradiated clone A2b or control Z1a in the ears and 48 hrs later DTH reactivity was measured by the degree of swelling.

Table 1   Detection of anti-idiotypic and anti-activated T cell

responses in vaccinated individuals using varius stimulator T cells.

| Stimulator T cells | | Response of vaccinated individual's T cells |
|---|---|---|
| Bear idiotype of T cell vaccine | Activated | |
| Yes | No | Anti-idiotypic |
| No | Yes | Anti-activated |
| Yes | Yes | Combined anti-idiotypic and anti-activated |
| No | No | Negative control (background) |

Table 2   Immunological specificity of anti-idiotypic helper and

suppressor clones induced to T cell vaccination.

| Anti-idiotypic | | Effect of anti-idiotypic clone on T cell proliferation (cpm x $10^{-3}$) | | | |
|---|---|---|---|---|---|
| clone | Phenotype | Z1a alone | A2b alone | Z1a +BP | A2b +MT |
| None | - | 8+0.2 | 9+2 | 99+12 | 92+1 |
| TR1 | Helper (CD4$^+$) | 26+3 | 12+1 | 83+6 | 106+9 |
| TR8 | Suppressor (CD*$^+$) | 4+1 | 5+1 | 49+4 | 82+10 |

Clones TR1 and TR8 were isolated from the popliteal lymph nodes of Lewis rats that had been vaccinated against EAE with $10^4$ Z1a clone cells. TR1 was found to be helper T cell (W3/25[+], OX8[−]) and TR8 was found to be a suppressor T cell (W3/25[−], OX8[+]). $10^4$ cells of each clone were irradiated to prevent their proliferation (1500R) and added to $2.5 \times 10^4$ cells of specific clone Z1a or control clone A2b, either alone or with their respective antigens: myelin basic protein (BP) or Mycobacterium tuberculosis (MT). Antigen presenting cells for the thymus ($10^5$) were added after being irradiated (1500R). The proliferative responses of Z1a or A2b were measured 3 days later by the incorporation of tritiated thymidine into DNA.

Table 3  Anti-idiotypic DTH is specific for effective T cell vaccination

| A2b vaccine | Resistance to AA | DTH ear swelling x $10^{-2}$mm at 48 hr to clone A2b |
|---|---|---|
| None | None | 8+1 |
| Irradiated | None | 10+1 |
| Glutaraldehyde | Yes | 25+2 |

Lewis rats were inoculated with clone A2b ($2 \times 10^7$) treated with irradiation (1500R) or with glutaraldehyde (0.3%, 15 minutes. Only the latter vaccination induces resistance to AA (1). Two weeks later the rats were treated for DTH reactivity to irradiated A2b cells ($10^5$).

Table 4  Non-activated T cell clones elicit an anti-idiotypic response; activated T cell clones elicted an anti-activated indicator response.

| T cells used to elicit DTH ear swelling in rats vaccinated with clone A2b | DTH reactivity mm x $10^{-2} \pm$ SE |
|---|---|
| non-activated A2b | 10 + 1.3 |
| non-activated D9 | 2.5 + 1 |
| activated A2b | 33 + 6 |
| activated D9 | 27 + 7 |

## Claims

1. An in vitro diagnostic assay for determining the efficacy of a vaccination against an autoimmune disease administered to a patient in need thereof in the form of a vaccine comprising suitably treated T cells, membranes of suitably treated T cells or fragments thereof, which assay comprises withdrawing peripheral blood cells of the patient for use in a proliferative assay, determining either one or both of the anti-idiotypic T cell response of such peripheral cells and/or the anti-activated indicator cell response to activated T cells of such peripheral cells, the degree of response being indicative of the efficacy of the vaccination.

2. The assay according to claim 1, which is a proliferation test in which the anti-idiotypic response is determined.

3. The assay according to claim 1, which is a proliferation test in which the anti-activated indicator response is determined.

4. The assay according to any one of claims 1 to 3 in which the assay on the peripheral blood cells is performed with activated T cells, T cell membranes or T cell membrane fragments obtained form the patient before vaccination or from another suitable source.

5. The assay according to any one of claims 1 to 3 in which the assay on the peripheral blood cells of the patient is performed with activated T cell membranes or membrane fragments obtained from a source other than the patient.

6. A kit for determining the efficacy of a vaccination against an autoimmune disease administered to a patient in need thereof in the form of a vaccine comprising suitably treated T cells, membranes of suitably treated T cells or fragments thereof, which kit comprises the effective ingredients required for carrying out said determination in a form suitable for prolonged storage, diluting means and means for evaluating the results of the measurement.

7. The kit according to claim 6 in which the components are provided in unit dosage form.

8. The kit according to claim 6 or 7 for use in in vitro or in vivo diagnostic assays for determining the efficacy of a vaccination against an autoimmune disease administered to a patient in need thereof in the form of a vaccine comprising suitably treated T cells, membranes of suitably treated T cells or fragments thereof.

Fig. 1

0 291 046

0 291 046

Fig. 2

Fig. 3

0 291 046

## DOCUMENTS CONSIDERED TO BE RELEVANT

| Category | Citation of document with indication, where appropriate, of relevant passages | Relevant to claim | CLASSIFICATION OF THE APPLICATION (Int Cl.4) |
|---|---|---|---|
| A | US - A - 4 402 934 (M.C. TEO-DORESCU et al.)<br><br>* Abstract; claims 1-10 * | 1,6 | G 01 N 33/80<br>G 01 N 33/564 |
| D,P, A | PROCEEDINGS OF THE NATIONAL ACADEMY OF SCIENCES OF THE UNITED STATES OF AMERICA, vol. 84, no. 13, July 1987<br>OFER LIDER et al. "Therapeutic vaccination against adjuvant arthritis using autoimmune T cells treated with hydrostatic pressure"<br>pages 4577-4580<br><br>* Abstract * | 1 | |
| D,A | US - A - 4 634 590 (I.R. COHEN et al.)<br><br>* Abstract; claims * | 1 | **TECHNICAL FIELDS SEARCHED (Int. Cl.4)**<br><br>G 01 N 33/00<br>A 61 K 35/00 |
| D,P, A | EP - A2 - 0 261 648 (YEDA RESEARCH AND DEVELOPMENT COMPANY, LTD.)<br><br>* Abstract * | 1 | |

The present search report has been drawn up for all claims

| Place of search | Date of completion of the search | Examiner |
|---|---|---|
| VIENNA | 29-07-1988 | SCHNASS |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82